# EUROPEAN PATENT SPECIFICATION

(11) **EP 0 383 591 B1**
(45) Date of publication and mention of the grant of the patent: **21.11.2001**
(21) Application number: 90301607.9
(22) Date of filing: 15.02.1990
(51) Int. Cl.: A61K 31/44

(54) **Use of 5-methyl-1-phenyl-2-(1H)-pyridone in the prevention of fibrotic lesions**
Verwendung von 5-methyl-1-phenyl-2-(1H)-pyridon zur Vorbeugung fibrotischer Schädigungen
Utilisation de la 5-méthyl-1-phényl-2-(1H)-pyridone pour la prévention des lésions fibrotiques

(30) Priority: 15.02.1989 JP 3543689
(43) Date of publication of application: 22.08.1990
(60) Divisional application: 01107235.2
(73) Proprietor: Yamauchi, Shitotomo, Chiyoda, Tokyo 102 (JP); MARGOLIN, Solomon B., Dallas, Texas 75225 (US)
(72) Inventor: Margolin, Solmon B., Dallas, Texas 75225 (US)
(74) Representative: Bannerman, David Gardner

(56) References cited:
- US-A- 4 042 699
- FEDERATION OF AMERICAN SOCIETIES FOR EXPERIMENTAL BIOLOGY, FED. PROC., New Orleans, Louisiana, 15th - 23rd April 1982, vol. 41, no. 5, abstract no. 7480; S. MARGOLIN et al.: "Removal of interstitial Pulmonary fibrosis (asbestos-induced) by oral chemotherapy with pirfenidone"
- DRUGS OF THE FUTURE, vol. 11, no. 6, June 1977, pages 396-397; P.J. ROBERTS et al.: "Pirfenidone"

## Description

The present invention relates to the use of a medical composition for the prevention reparation of fibrotic lesions.

Herein, the term "anti-fibro", "anti-fibrotic" or "anti-fibrosis" refers to preparation from pathological polymerization of collagen in lung fibrosis, arteriosclerosis, prostatic hypertrophy, keloid, myocarditis, collagen disease, scar, wrinkle, etc., and reparation as well as normalization of the existing pathological fibrotic tissues.

5-methyl-1-phenyl-2-(lH)-pyridone itself is a known compound and its pharmacological effects are disclosed, for example, in Japanese Patent Application KOKAI (Laid-Open) Nos. 87677/1974 and 128438/1976 as an anti-inflammatory agent including antipyretic and analgesic effects. However, it has been discovered by the present inventors that the compound has an anti-fibrotic activity. Heretofore, no effective pharmacological agent or composition has been available for the prevention or removal of pathologic fibrotic lesions of the lungs, prostate glands, muscoloskeletal diseases, myocardial degeneration, myocardial infarction, arteriosclerosis, and other lesional fibrosis.

For example, powerful anti-inflammatory glucocorticoids (hormones relating to carbohydrate metabolism) such as hydrocortisone or prednisolone administered in very large doses have repeatedly been shown to be ineffective against fibrotic disease. These glucocorticoids do not arrest or remove such life-threatening fibrotic lesions. The glucocorticoids may be are effective, however, as anti-inflammatory agents under such conditions that they may temporarily ameliorate the secondary acute inflammation flare-ups which intermittently occur in tissues or organs damaged by fibrotic disease. Indeed, excessive and prolonged administration of glucocorticoids in pulmonary fibrotic disease may cause destruction of tissues due to fibrosis or an exacerbation and acceleration of the fibrotic destruction.

Antopol (1950) was the first of many investigators who found that the anti-inflammatory glucocorticoids readily enhance fibrotic degeneration of lung tissues. Similarly, the non-steroidal anti-inflammatory agents such as aspirin, salicylates, phenylbutazone, indomethacin, various phenylacetic acid derivatives and the like have also failed to arrest formation of, or cause repair of the progressive, chronic fibrotic damage to lung tissues, prostatic tissues, musculoskeletal tissues, etc.

The present inventors have discovered that 5-methyl-1-phenyl-2-(1H)-pyridone exerts heretofore unknown pharmacological properties which are useful in the medicinal therapy of fibrotic disease and based on the finding, they have developed drugs comprising the compound as the active ingredient which are effective for the reparation and prevention of fibrotic lesional tissues.

According to one aspect of the invention there is provided the use of 5-methyl-1-phenyl-2-(1H)-pyridone for the manufacture of a medicament for the prevention of fibrotic lesions.

According to another aspect of the invention there is provided the use of 5-methyl-1-phenyl-2-(1H)-pyridone for the manufacture of a medicament for the prevention of fibrotic lesions wherein the lesions are associated with pulmonary fibrosis.

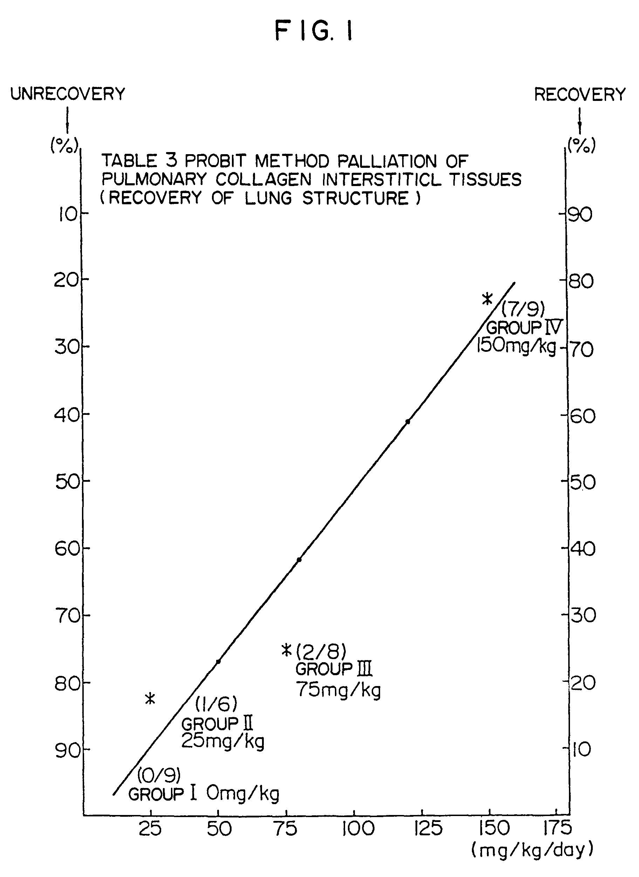

Figure 1 is a graph representing the animal test data shown in Table 3 indicating the effect of the drug used in the present invention.

The "anti-fibrotic" activity described herein differs from "fibrinolytic" or "anti-fibrin" activity. The "fibrinolvtic" or "anti-fibrin" activity refers to the biological ability of a pharmaceutical substance to (1) prevent fibrin formation (prevent formation of a blood clot) or (2) lyse or dissolve a previously-formed blood clot.

The "anti-fibrotic" activity discovered by the present inventors and as used herein refers to the ability of an active substance to (1) prevent an excessive pathologic accumulation of collagenous scar or connective tissue in various body structures and organs (usually triggered by some injury, allergy, infection, or by some inherited genetic aberration), or (2) cause the non-surgical removal or biological dissolution of an existing excessive and pathologic accumulation of fibrotic collagenous tissue (for example, as in the dissolution of life-threatening fibrotic lesions of the lung found in patients with asbestosis).

### A. CONNECTIVE TISSUE PROTEINS OF MAMMALS

Three major classifications of connective tissue proteins are recognized with the largest portions consisting of collagen types (70 to 80%) and elastin types (15 to 20%). A miscellaneous group constitutes the third and smallest class.

The general biochemical characteristics of the collagen types which constitute the principle protein (1) in normal white connective tissue and (2) in scar or fibrotic tissue, are summarized in Table 1, as contrasted with elastin types. For example, collagen is sparingly soluble in water, but readily converted to water soluble gelatin upon boiling in an acid or alkali. In contrast, the highly water soluble elastin does not convert to gelatin upon boiling in an acid or alkali.

The elastin constitutes the principle protein of yellow connective tissue found in elastic structures such as the walls of larger blood vessels and walls of lung alveoli.

Investigations on the molecular biochemical level of tissues have demonstrated a very slow turnover rate for metabolic processes involving fibrotic lung collagen. In fact, the metabolic rate is measured in years. By contrast, the metabolic rates of the other connective tissue collagens including elastin and the like are measured and expressed in hours and days (White, Handler and Smith, 1973, page 983).

### B. INTERSTITIAL PROLIFERATION (HYPERPLASIA) OF FIBROBLAST-TYPE CELLS IN LUNGS AND OTHER ORGAN TISSUES

The synthesis of various collagens found in scar or fibrotic structures takes place in fibroblast cells which then extrude the collagen into the surrounding matrix. During this wound repair process, there are (1) a rapid proliferation and increase in the number of fibroblasts at the site, and (2) a sharp rise in the rate of the synthesis and extrusion of collagen. If these two phenomena are not prevented, the pathologic and progressive accumulation of collagen would cause polymerization and fibrotic disease (for example, impairment of respiratory function, impaired circulatory function via fibrotic changes in arterial walls, fibrotic degeneration of renal and liver function, degenerative musculoskeletal function, fibrotic degeneration of cardiac muscle or skeletal muscle, fibrotic degenerative changes in neuronal tissues in the central nervous system as well as the peripheral nervous system, etc.) [Kinichiro Kajikawa, "Concept of Collagen Disease", 52 (1972)].

With pulmonary interstitial fibrotic hyperplasia, small and firm nodules are palpable throughout the lung tissue, and upon gross examination are recognized from their opaque, airless structure to be foci, of abnormal accumulations of fibrotic connective tissue. Such foci vary in size and color according to their age. Their aggressive and continued enlargement and coalescence ultimately leads to collagenous solidification of large segments of the lungs.

These enlarging foci also impinge upon the lung capillaries thereby to reduce pulmonary blood flow, and at the same time, impede lymphatic drainage from the lungs. As a consequence, exudate accumulates within the alveoli, and secondary thickening of the alveolar wall ensues. These interacting processes sharply reduce the efficiency of the gaseous exchange in the lung alveoli, which is a primary function of the normal lung.

In addition, these pulmonary fibrotic alternations and accumulations raise the pulmonary blood vessel resistance and lead to cor pulmonale (sharply elevated pulmonary blood pressure). Prolonged elevated pulmonary blood pressure almost invariably leads to congestive heart failure in addition to the cyanosis caused by inadequate pulmonary exchange of oxygen and carbon dioxide. The prognosis is poor and the incidence of severe morbidity and deaths is high.

Furthermore, the fibrosis of the lung impairs the physiological and biochemical functions of the lung that are independent of the pulmonary gas exchange (oxygen and carbon dioxide) role of the lungs cited above. These include:
(1) filtration, degradation and removal of the following substances:
   (a) aged leucocytes from the blood, and
   (b) particulate matter (for example, tissue cell debris, blood cell aggregates, inert foreign matter, small thrombi);
(2) synthesis of adequate supplies of heparin.

Heparin is a useful substance that normally prevents the formation of life-threatening blood clot in the major blood vessels (for example, cerebral and coronary blood vessels).

### C. DIFFERENTIATION BETWEEN ANTI-FIBROTIC ACTIVITY AND ANTI-INFLAMMATORY ACTIVITY

Pharmacological anti-fibrotic activity as exemplified by the arrest and removal of lung scarring (interstitial hyperplasia and fibrotic foci), or pathologic fibrotic lesions in other organs and tissues described herein, is clearly distinct from and independent of any pharmacological anti-inflammatory activity.

The debilitating pathologic degeneration of organs and tissues affected by fibrotic disease continues for extended periods of time, measured in months or years, beyond the short-term (hours and days) of exacerbating inflammatory flare-ups (classical clinical and histophathological signs of edema, local heat and leucocytic infiltration have disappeared).

The composition of this invention is effective for treatment of disease caused by the pathologic and excessive fibrotic accumulations such as pulmonary fibrosis, benign prostate hypertrophy, coronary infarcts, cerebral infarcts, myocardiac fibrosis, musculoskeletal fibrosis, post-surgical adhesions, liver cirrhosis, renal fibrotic disease, fibrotic vascular disease (atherosclerosis, varix or varicose veins), scleroderma, Alzheimer's disease, diabetic retionopathy, glaucoma, etc. 5-Methyl-1-phenyl-2-(1H)-pyridone (5MP2P) not only arrests the formation of new fibrotic tissue but causes removal of previously formed fibrotic collagen-containing tissue. These pharmacological properties of 5MP2P are heretofore unknown.

This has been repeatedly demonstrated in experimentally induced pulmonary fibrosis, and in humans with advanced life-threatening pulmonary fibrosis.

The present application describes a method for arresting formation of or causing a removal of a pathogenic accumulation of water-insoluble collagenous connective tissue (for example, excessive scar or lesional fibrotic tissue, etc.). By medicinally removing such pathologic collagenous tissue in fibrotic lungs, 5MP2P eliminates or prevents:
(1) the mechanical compression or occlusion (stenosis) of blood vessels (for example, pulmonary arteries, veins, and capillaries), pulmonary bronchioles and alveoli.
(2) the inhibition of the primary respiratory function of the alveoli of the lungs, namely, the exchange of oxygen (O₂), and carbon dioxide (CO₂) gases.
(3) the increased pulmonary blood vessel resistance (cor pulmonale) which readily causes fatal congestive heart failure because of the excessive workload on cardiac muscle that is engendered by the cor pulmonale.

The dramatic and novel pulmonary anti-fibrotic activity by 5MP2P has been observed and demonstrated in laboratory animal experiments (rats, hamsters, dogs) and humans. The anti-fibrotic activity in cardiac infarctions, benign prostatic hypertrophy, and post-operative adhesions has been observed in humans. The renal anti-fibrotic activity has been demonstrated in hamsters. In every instance, the anti-fibrotic activity was clearly distinct from any anti-inflammatory properties.

The acute toxicity of the ingredient in the medical composition of the present invention which exerts the anti-fibrotic activity is as shown in the table below.

| Acute Toxicity (LD₅₀ : mg/kg) | | | | |
|---|---|---|---|---|
| Route for Administration | | | | 10% Ointment |
| Animal | p.o. (number) | i.v. (number) | i.p. (number) | p.o. (number) |
| Mouse | 997.7 (40) | 285± 5 (50) | 600±43 (60) | 11,500±1,100 (43) |
| Rat male: | 1,295 (25) | | 430±29 (42) | 12,500 (10) |
| | | | | |
| female: | 2,300 (30) | | | |
| Guinea pig | 810±25 (30) | | 460±28 (25) | |
| Rabbit | | 280±32 (12) | | |
| Cat | 500 (17) | 40 (4) | | |
| Dog | 300 (11) | 200 (6) | | |
| Monkey | | 100 (3) | | |

Hereafter the present invention is described below by referring to examples below.

The anti-fibrotic activity measured against pulmonary fibrosis was found to be quite dissimilar to and independent of anti-inflammatory activity when these activities were assayed in rats and hamsters. Experiments in dogs and human clinical trials affirm these findings. 5MP2P has been extensively studied for oral anti-fibrotic activity in laboratory animals and in humans. The anti-fibrotic effect in pulmonary fibrosis was demonstrated upon oral administration:
(1) in diets or by gavage to rats or hamsters,
(2) oral capsules in dogs, and
(3) oral administration to humans.

### Example 1

The results of a histopathological examination of the lungs of rats for fibrosis (interstitial hyperplasia) after receiving 300 mg/kg body of 5MP2P in the diet for three months are summarized in Table 2. The individual microscopic readings of the lung are also shown in Table 2, a score schedule of 0, 1, 2 and 3 reflects the degree of fibrosis.

The data in Table 3 reveal a statistically significant reduction in amount of fibrosis in rats receiving 5MP2P as compared to placebo control rats (Group 1). The mean score for the controls (Group 1) was 1.63 + 0.23, and for Group IV (5MP2P, 300 mg/kg body weight daily) was 0.95 + 0.23.

Student's T value was 2.43, with P less than 0.02 (highly significant statistically).

In male and female Beagle dogs, the anti-fibrotic activity was found to be a direct function of the dosage of 5MP2P administered, a classical pharmacological dose-response (Table 3, Fig. 1). Lung tissues examined microscopically, and scored on a schedule of 0, 1, 2 and 3 for fibrosis resulted in clear demonstration of a statistically significant reduction in pulmonary fibrosis in dogs given the drug as compared to control animals.

The mean score for Group I (Control) was 2.11 + 0.31, and for Group IV, which received 5MP2P, 150 mg/kg per day orally in capsules, was 0.22 + 0.15.

In hamsters, pulmonary fibrosis induced with crysotile asbestos was removed following oral 5MP2P (cf. Table 4).

This anti-fibrotic activity was not simply a palliative (relieving) effect.

The asbestos-induced fibrosis did not recur after the 5MP2P had been discontinued for two months.

A similar experience has been observed in trials on human patients with pulmonary fibrosis caused by asbestos.

For the first time ever, 5MP2P makes possible a pulmonary resolution process whereby a life-threatening solidified fibrotic lung disease can be restored to a relatively normal tissue where the alveoli are no longer collapsed or occluded.

That is, the microscopic examination reveals that the tissues are regenerated and become normal, spongy lungs.

The novel role of 5MP2P in the therapeutic repair of fibrotic lung tissue featuring removal of fibrotic lesions, and concomitant regeneration of normal lung tissue has been observed in experimental asbestosis by histopathological examination of lung tissue specimens under the light microscope, and electron microscopy (Table 4).

Very little, if any, fibrotic alterations are seen after treatment with adequate doses of 5MP2P.

A further novel discovery was the demonstration under the electron microscope that the lung cell-imbedded asbestos fibers which had initiated and maintained the extensive fibrotic lesions also had been removed. This was subsequently confirmed by ashing of lung specimens in a laboratory oven, and then determining the asbestos content.

The discovery of this additional novel "clearing" property of 5MP2P, for the first time, affords a therapeutic pharmacological remedy for chronic respiratory disease caused accumulation in the lungs by inhalation of harmful foreign matter polluted air, asbestos, black lung of miners, silicosis, industrial dust (grain, lime, fertilizers, cotton fibers, glass fibers, plastics, etc.). However, such uses of 5MP 2P are outside the scope of the present invention.

**Table 1**

| Contrast Between Properties of Collagen and Elastin | | | |
|---|---|---|---|
| | Property | Collagen | Elastin |
| 1. | Water soluble | - | + |
| 2. | Converts to gelatin on boiling | + | - |
| 3. | Primarily in white connective tissue | + | - |
| 4. | Primarily in yellow connective tissue | - | + |
| 5. | Primarily associated with highly elastic structures (e.g., blood vessels) | - | + |
| 6. | Primarily in organ structural tissue; fibrotic or scar tissue (e.g., lung fibrosis, etc.) | + | - |
| 7. | Metabolic turnover rate | low | high |

**Table 2**

| Group I (control) | | | | | |
|---|---|---|---|---|---|
| | | Lung Connective Tissue Score | | | |
| Animal Number | Sex | 0 | 1 | 2 | 3 |
| 104 | F | | | | X |
| 8 | M | | | X | |
| 72 | F | X | | | |
| 74 | F | | | | X |
| 75 | F | | X | | |
| 80 | F | | | | X |
| 81 | F | | | | X |
| 82 | F | | X | | |
| 88 | F | | | X | |
| 94 | F | | X | | |
| 1 | M | | X | | |
| 19 | M | | X | | |
| 26 | M | | | X | |
| 36 | M | | X | | |
| 43 | M | | | | X |
| 45 | M | X | | | |
| 52 | M | | X | | |
| 53 | M | | | X | |
| 55 | M | | X | | |
| Total | | 2 | 8 | 4 | 5 |
| Mean | 1.63 | | | | |
| S.E. | 0.23 | | | | |
| 95 | F | | X | | |
| 86 | F | | | | X |
| 93 | F | | X | | |
| 97 | F | X | | | |
| 98 | F | | X | | |
| 99 | F | | X | | |
| 119 | F | | X | | |
| 122 | F | | X | | |
| 123 | F | X | | | |
| 135 | F | X | | | |
| 5 | M | | X | | |
| 11 | M | | X | | |
| 16 | M | | X | | |
| 29 | M | X | | | |
| 31 | M | X | | | |
| 32 | M | | X | | |
| 34 | M | | X | | |
| 35 | M | | | X | |
| 40 | M | | | X | |
| Total | | 5 | 11 | 2 | 1 |
| Mean | 0.95 | | | | |
| S.E. | 0.18 | | | | |
| t | 2.43 | | | | |
| P | <0.02 | | | | |

**Table 3**

| Effect of Oral 5MP2P upon Pulmonary Interstitial Hyperplasia (Fibrosis) in Dogs | | | | | | | |
|---|---|---|---|---|---|---|---|
| | | Hyperplasia Scores | | | | | |
| Group | Number of Dogs | 0¹⁾ | 1 | 2 | 3 | Average Scores | Incidence of Normal Lung |
| I. Control (0.0%) | 9 | 0 | 3 | 2 | 4 | 2.11+0.31 | 0/9 |
| II. 5MP2P | 6 | 1 | 1 | 4 | 0 | 1.50+0.34 | 1/6 |
| (16.7%) 25 MG/KG/day | | | | | | | |
| III. 5MP2P (25.0%) 75 MG/KG/day | 8 | 2 | 2 | 3 | 1 | 1.38+0.38 | 2/8 |
| IV. 5MP2P 150 MG/KG/day | 9 | 7 | 2 | 0 | 0 | 0.22+0.15** | 7/9 |

| | | | | | | | |
|---|---|---|---|---|---|---|---|
| 1) Degree of hyperplasia (fibrosis) 0 = normal tissue 1 = minimal 2 = moderate 3 = severe | | | | | | | |
| ** Highly Statistically Significant (P 0.001) | | | | | | | |

**Table 4**

| Effect of Oral 5MP2P upon Asbestos-Induced Pulmonary Interstitial Fibrosis in Hamsters | | | | |
|---|---|---|---|---|
| | | Pulmonary Fibrosis Scores | | |
| Group | Animal Number | Lung Density | Light Microscope | Electron Microscope |
| I. Control | 1 | 0.95 | 0 | 0 |
| (No Asbestos (-); | 2 | 0.90 | 1 | 0 |
| No 5MP2P) | 3 | 1.05 | 1 | 1 |
| | 4 | 1.10 | 0 | 0 |
| Average | | 1.00+0.05 | 0.50+0.25 | 0.25+0.25 |
| II. Asbestos (+); | 5 | 2.70 | 3 | 3 |
| No 5MP2P (-) | 6 | 1.90 | 2 | 3 |
| | 7 | 2.53 | 3 | 2 |
| | 9 | 2.98 | 3 | 3 |
| Average | | 2.53+0.23** | 2.75+0.25** | 2.75+0.25** |
| III. Asbestos | 10 | 0.98 | 0 | 0 |
| (+) ²⁾; | 12 | 1.04 | 2 | 1 |
| Plus 5MP2P (+) | 13 | 1.26 | 1 | 0 |
| | 14 | 1.41 | 1 | 0 |
| Average | | 1.17+0.10** | 1.00+0.41** | 0.25+0.25** |

| | | | | |
|---|---|---|---|---|
| Student's "T" Values: Group II VS III ····· 5.9** 3.7** 7.1** Group II VS I ····· 6.5** 5.9** 7.1** Degree of Fibrosis: 0 = normal tissue 1 = minimal 2 = moderate 3 = severe 2) Asbestos by inhalation for 5 days; 5MP2P, 500 MG/KG/day, orally in the diet for two months, beginning two months after the five-day exposure to asbestos dust. ** Highly Statistically Significant (P<0.001) | | | | |

Examples of medical preparations include:
(1) capsules, (2) tablets, (3) powders, (4) granules, (5) syrups, (6) injection (intravenous, intramuscular or drip administration), (7) cream, (8) ointment, (9) inhalation, (10) eye drop, (11) (12) suppositories, (13) pills, etc.

The above preparations are available. Among them, capsules, injections, cream and ointments are preferred preparations. Medical preparations generally also comprise one or more pharmaceutically suitable carriers.

### Test Example 1

In one capsule, 100 mg, 200 mg or 400 mg of 5MP2P is contained.

### Test Example 2

5% or 10% hydrophilic ointment of 5MP2P.

5MP2P is usually administered to human in a dose of 100 to 800 mg/time in America; it is considered to be appropriately in a daily dose of 400 to 2,400 mg in total for 3 administrations a day.

## Claims

1. Use of 5-methyl-1-phenyl-2-(1H)-pyridone for the manufacture of a medicament for the prevention of fibrotic lesions.

2. Use according to claim 1 wherein the lesions are associated with pulmonary fibrosis.

3. Use of 5-methyl-1-phenyl-2-(lH)-pyridone for the manufacture of a medicament for the arrest, repair, reparation and/or prevention of fibrotic lesions associated with: the prostate glands including benign prostatic hypertrophy; coronary infarcts, cerebral infarcts, myocardiac fibrosis; myocardial infarction, myocardial degeneration, myocarditis, muscoloskeletal fibrosis, post-surgical adhesions, liver cirrhosis, renal fibrotic disease, fibrotic vascular disease, arteriosclerosis, scleroderma, Alzheimer disease, diabetic retinopathy, glaucoma, keloid, collagen disease or scar wrinkle.

4. Use according to any of claims 1 to 3 in which said 5-methyl-1-phenyl-2-(1H)-pyridone is present in a hydrophilic ointment in an amount of from 5 to 10%.

## Patentansprüche

1. Verwendung von 5-Methyl-l-phenyl-2-(1H)-pyridon zur Herstellung eines Medikaments zur Vorbeugung fibrotischer Schädigungen.

2. Verwendung gemäß Anspruch 1, wobei die Schädigungen mit Lungenfibrose in Verbindung stehen.

3. Verwendung von 5-Methyl-1-phenyl-2-(1H)-pyridon zur Herstellung eines Medikaments zur Hemmung, Reparatur, Regeneration und/oder Vorbeugung fibrotischer Schädigungen, die verbunden sind mit: den Prostatadrüsen einschließlich einer gutartigen Prostatahypertrophie; Koronarinfarkten, Hirninfarkten, Myokardfibrose; Myokardinfarkt, Herzmuskelentartung, Myokarditis, Skelettmuskulaturfibrose, postoperativen Verwachsungen, Leberzirrhose, Nierenfibrosekrankheit, fibrotischem Gefäßleiden, Arteriosklerose, Sklerodermie, Alzheimer-Krankheit, diabetischer Retinopathie, Glaukom, Keloid, Kollagenose oder Narbenfalten.

4. Verwendung gemäß einem der Ansprüche 1 bis 3, wobei das 5-Methyl-1-phenyl-2-(1H)-pyridon in einer hydrophilen Salbe in einer Menge von 5-10 % vorliegt.

## Revendications

1. Utilisation de 5-méthyl-phényl-2-(1H)-pyridone, pour la fabrication d'un médicament pour empêcher les lésions fibrotiques.

2. Utilisation selon la revendication 1, dans laquelle les léions sont associés à de la fibrose pulmonaire.

3. Utilisation de 5-méthyl-phényl-2-(1H)-pyridone, pour la fabrication d'un médicament pour faire cesser, réparer, assurer la réfection et/ou la prévention des lésions fibrotiques associées : aux glandes prostatiques y compris une hypertrophie prostatique bénigne, aux infarctus coronaires, aux infarctus cérébraux, à la fibrose myocardique ; à l'infarctus myocardique ; à la dégénérescence myocardique ; à la fibrose musculosquelettique, aux adhérences post-chirurgicales, à la cirhose du foie, aux troubles fibrotiques rénaux, aux troubles vasculaires fibrotiques, à l'artériosclérose, à la sclérodermie, à la maladie d'Alzheimer, à la retinopathie diabétique, au glaucome, la chéloïde, la maladie du collagène ou le plissement cicatriciel.

4. Utilisation selon l'une quelconque des revendications 1 à 3, dans laquelle ladite 5-méthyl-phényl-2-(1H)-pyridone est présente dans une pommade hydrophile, en une quantité comprise entre 5 et 10 %.
